# EUROPÄISCHE PATENTSCHRIFT

(11) **EP 1 312 307 B1**
(45) Veröffentlichungstag und Bekanntmachung des Hinweises auf die Patenterteilung: **07.09.2005**
(21) Anmeldenummer: 02102560.6
(22) Anmeldetag: 12.11.2002
(51) Int. Cl.: A61B 5/055

(54) **Mammographie-Zusatz für MR-Elastographie**
Mammography accessory for magnetic resonance (MR) elastography
Accessoire de mammographie pour élastographie par résonance magnétique

(30) Priorität: 15.11.2001 DE 10156178
(43) Veröffentlichungstag der Anmeldung: 21.05.2003
(73) Patentinhaber: Philips Intellectual Property & Standards GmbH, 20099 Hamburg (DE); Koninklijke Philips Electronics N.V., 5621 BA Eindhoven (NL)
(72) Erfinder: Sinkus, Dr. Ralph, 52088, Aachen (DE); Dargatz, Michael, 52088, Aachen (DE); Kuhl, Dr. Christiane, 52088, Aachen (DE)
(74) Vertreter: Volmer, Georg

(56) Entgegenhaltungen:
- DE-A- 19 952 880
- US-A- 5 952 828
- US-A- 6 037 774

## Beschreibung

Die Erfindung betrifft einen Mammographie-Zusatz für MR-Elastographie (MR = Magnetresonanz) sowie auf ein MR-Gerät mit einem Mammographie-Zusatz.

Bei der MR-Elastographie wird die Tatsache ausgenutzt, dass die Phase in einem MR-Bild des Untersuchungsobjektes sich infolge von mechanischen Wellen ändert, die auf das Untersuchungsobjekt einwirken. Das Ausmaß dieser Änderung hängt von der Auslenkung (d. h. der Verschiebung aus der Ruhelage) des Gewebes infolge der mechanischen Wellen ab. Somit lässt sich aus den MR-Phasenbildern, d. h. Bildern, die die Phase der Kernmagnetisierung darstellen, Information über bestimmte mechanische Parameter des Gewebes ableiten, z. B. über die Elastizität.

Ein bevorzugtes Anwendungsgebiet der MR-Elastographie ist die Mammographie, d. h. die Darstellung der weiblichen Brust (Mamma). Ein dafür geeigneter Mammographie-Zusatz ist aus der US-Appln 09/743659 (PHD 99 -152) bekannt, bei dem durch einen horizontal und senkrecht zur Längsrichtung der zu untersuchenden Patientin schwingenden Schwingungsapplikator in der Mamma longitudinale mechanische Wellen angeregt werden. Ein Nachteil ist die lange Dauer einer mit diesem Zusatz durchgeführten Untersuchung (bis zu 30 Minuten). Den gleichen Nachteil weist die Anordnung nach der US-PS 5,952,828 auf. Die Schwingungsapplikatoren wirken dabei von unten auf die Mammae der bäuchlings gelagerten Patientin ein, wobei sie eine tangential zu ihrer Kontaktfläche verlaufende Schwingung ausführen. Dadurch werden in den Mammae im wesentlichen aber nur transversale Wellen angeregt, die eine geringere Eindringtiefe haben als longitudinale Wellen.

Es ist Aufgabe der vorliegenden Erfindung, einen Mammographie-Zusatz zu schaffen, mit dem die Untersuchungszeit reduziert werden kann und der in den Mammae überwiegend longitudinale mechanische Wellen anregt.

Diese Aufgabe wird erfindungsgemäß gelöst durch einen Mammographie-Zusatz für Elastographie mit einer Lagerungs-Einheit, die eine mit wenigstens einer Öffnung zur Aufnahme wenigstens einer Mamma versehene Stützfläche aufweist und mit einer Schwingungserzeuger-Anordnung zur Erzeugung von mechanischen Wellen über wenigstens einen in Längsrichtung einer zu untersuchenden Patientin hin- und herschwingenden Schwingungsapplikator mit einer zu seiner Schwingungsrichtung zumindest näherungsweise senkrechten Kontaktfläche.

Ebenso wie bei den bekannten Mammographie-Zusätzen werden die Mammae in einem gewissen Umfang senkrecht zur Kontaktfläche der Schwingungsapplikatoren komprimiert. Während bei den bekannten Zusätzen die Kompression aber zur Folge hat, dass die Abmessungen der Mammae in der Längsrichtung der Patientin zunehmen, hat die Kompression der Mammae bei der Erfindung zur Folge, dass diese Abmessungen abnehmen, weil in diese Richtung die Kontaktfläche einwirkt. Die Abbildung der Mammae durch eine Anzahl von zur Längsrichtung senkrechten Schichtbildern (diese Orientierung der Schichtbilder ist notwendig, um gleichzeitig die Brustwand und die Achselbereiche abbilden zu können) erfordert eine geringere Anzahl von Schichten und damit eine geringere Zeit, um die für diese Schichten erforderlichen MR-Daten zu gewinnen.

Ein weiterer Vorteil ist, dass die Mammae in den einzelnen Schichten bei dieser Kompressionsrichtung weitgehend unverformt erscheinen. Bei den bekannten Mammographie-Zusätzen hingegen erscheinen die Mammae in diesen Schichtbildern seitlich oder von unten verformt, was die Diagnose erschwert und von vielen Untersuchern abgelehnt wird.

Die Ausgestaltung nach Anspruch 2 gestattet die Anpassung an unterschiedliche anatomische Verhältnisse. Die Kompression kann dabei so gewählt werden, dass einerseits die Mamma zwar fixiert ist und dass sie sich andererseits noch elastisch verformen kann. Die weitere Ausgestaltung nach Anspruch 3 gestattet die Fixierung der Mammae auch auf der Seite des Schwingungsapplikators. Der Schwingungsapplikator selbst kann dabei bezüglich der Lagerungs-Einheit ortsfest sein.

Grundsätzlich kann die Lagerungs-Einheit eine relativ breite Öffnung für die Aufnahme beider Mammae aufweisen. Die Ausgestaltung nach Anspruch 4 bietet demgegenüber den Vorteil, dass jede Mamma von einer MR-Empfangsspule umschlossen werden kann, was einen einwandfreien Empfang der MR-Signale ermöglicht. Darüber hinaus erlauben die nebeneinander angeordneten MR-Empfangsspulen Sense-Messungen, wodurch sich die Untersuchungszeit noch weiter verkürzt.

Für die Durchführung der MR-Elastographie ist es erforderlich, zeitgleich mit den mechanischen Wellen magnetische Gradientenfelder auf den Untersuchungsbereich einwirken zu lassen. Dies wird durch das MR-Gerät gem. Anspruch 5 sichergestellt.

Die Erfindung wird nachstehend anhand der Zeichnungen näher erläutert. Es zeigen:
- Fig. 1: ein MR-Gerät mit einem Mammographie-Zusatz,
- Fig. 2: den Mammographie-Zusatz in einer zur Längsrichtung senkrechten Schnittebene,
- Fig. 3: eine Detailansicht entlang der Linie A-A' in Fig. 2,
- Fig. 4a und 4b: einen Vergleich der Wirkung des erfindungsgemäßen Zusatzes mit dem bekannten Mammographie-Zusatz.

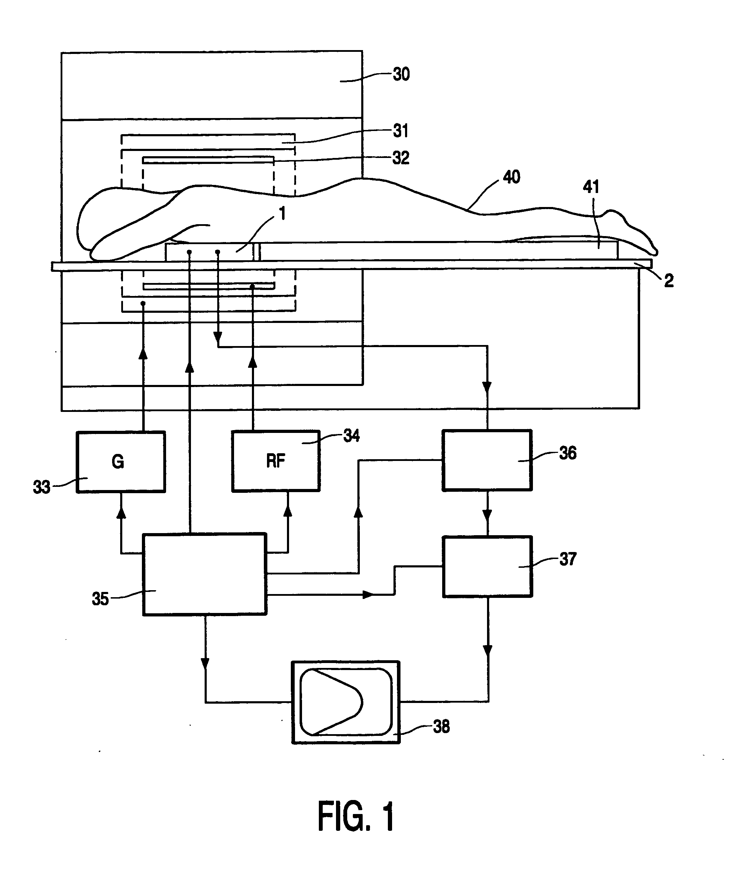

Fig. 1 stellt schematisch einen Querschnitt durch ein MR-Gerät dar und die zu seinem Betrieb erforderlichen Schaltungs- und Software-Komponenten. Das MR-Gerät umfasst einen Hauptfeldmagneten 30 zur Erzeugung eines horizontal und parallel zur Zeichenebene der Fig. 1 verlaufenden Hauptmagnetfeldes B0 Im Innern dieses zylinderförmigen Hauptfeldmagneten befindet sich ein Satz 31 von Gradientenspulen zur Erzeugung von Magnetfeldern mit Gradienten in drei zueinander senkrechten Richtungen. Im Innern dieser Gradientenspulen wiederum befindet sich eine Hochfrequenzspule 32 zur Erzeugung eines Hochfrequenzmagnetfeldes.

Die Ströme für die Gradientenspulen 31 werden von einer Generatoranordnung 33 geliefert, während die Ströme für die Hochfrequenzspule 32 von einem Hochfrequenzgenerator 34 geliefert werden. Der zeitliche Verlauf der von den Generatoren 33 und 34 erzeugten Ströme wird von einer Steuereinheit 35 vorgegeben und gesteuert. Die im Untersuchungsbereich erzeugten MR-Signale werde von einem MR-Empfänger 36 empfangen und aufbereitet und einer Bildverarbeitungseinheit 37 zugeführt, die aus den empfangenen MR-Signalen ein MR-Bild rekonstruiert, das auf einem Monitor 38 wiedergegeben wird.

Insoweit als bisher beschrieben, ist das MR-Gerät bekannt.

In dem durch die Spulen 31 und 32 definierten Untersuchungsbereich befindet sich eine Patientin 47, die auf einem Kissen 41 gelagert ist, das auf einem Patiententisch 2 aufliegt. Vor dem Kissen 41 ist ein Mammographie-Zusatz 1 angeordnet, der die gleiche Höhe hat wie das Kissen 41.

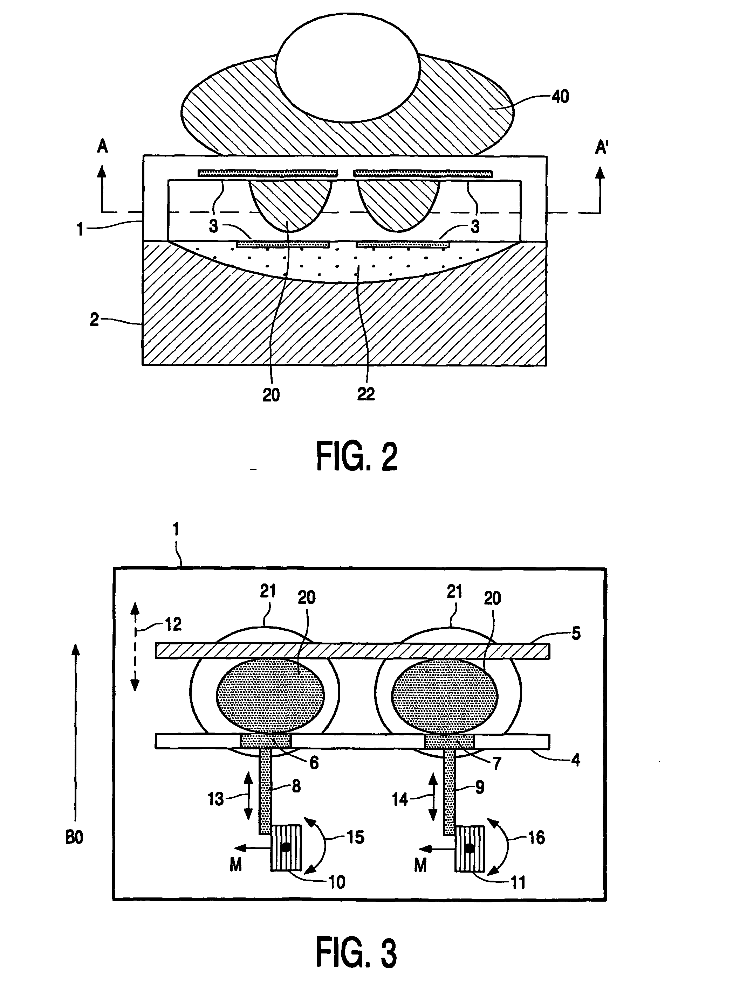

Der Mammographie-Zusatz ist in den Fig. 2 und 3 näher dargestellt, wobei Fig. 2 ihn in einer zu der Zeichenebene von Fig. 1 senkrechten vertikalen Schnittebene darstellt, während Fig. 3 eine Ansicht von unten zeigt, entlang der Linien A-A' in Fig. 2. Einige Komponenten sind der Einfachheit halber nur in einer der beiden Figuren dargestellt.

Wie Fig. 2 zeigt, umfasst der Mammographie-Zusatz eine brückenförmige Lagerungs-Einheit 1, deren Oberseite eine Stützfläche für die darauf in Bauchlage befindliche Patientin bildet. Die Stützfläche ist mit zwei nebeneinander liegenden Öffnung versehen, durch die hindurch die Mammae 20 der Patientin 40 frei nach unten hängen können. Auf ihrer Unterseite wird die Lagerungs-Einheit durch einen Kunststoffkörper 22 abgeschlossen, dessen Außenkontur der Muldenform der darunter befindlichen Tischplatte 2 des Patientenlagerungstisches angepasst ist.

Je zwei Spulen 3 befinden sich im oberen und unteren Bereich des Mammographie-Zusatzes in horizontalen Ebenen, wobei die oberen Spulen in die Stützfläche und die unteren Spulen in den Kunststoffkörper 22 integriert sind. Durch diese Anordnung ergibt sich ein homogenes räumliches Empfindlichkeitsprofil der Spulen. Das je einer Mamma zugeordnete Spulenpaar ist an einen gemeinsamen Empfangskanal angeschlossen. Da sich die Empfindlichkeitsprofile der nebeneinander angeordneten Spulen überlappen, sind zudem SENSE-Messungen in Rechts-Links-Richtung möglich, woraus sich ein zusätzlicher Zeitgewinn ergibt.

Gemäß Fig. 3 befinden sich im Innern des Mammographie-Zusatzes unterhalb der Öffnungen zwei Kompressionsplatten 4 und 5 in zur Längsrichtung des Patientenlagerungstisches senkrechten Ebenen. Die Kompressionsplatte 4 ist fest mit der Lagerungs-Einheit 1 verbunden, während die Kompressionsplatte 5 mit einer nicht näher dargestellten Mechanik auf die Kompressionsplatte 4 zu bzw. von ihr weg verstellbar ist. Dadurch kann die Kompression individuell an die Anatomie der jeweiligen Patientin angepasst werden.

Außerdem sind in der Lagerungs-Einheit zwei Schwingungserzeuger angebracht, die je einen Schwingungsapplikator enthalten. Jeder Schwingungsapplikator umfasst einen Stempel 6 bzw. 7 mit einer zur Tischlängsrichtung senkrechten Kontaktfläche und einen damit verbundenen, in Tischlängsrichtung verlaufenden Kolben 8 bzw. 9. Jeder Kolben führt in Richtung der Pfeile 13 bzw. 14 - also in seiner Längsrichtung - eine hin- und hergehende Schwingungsbewegung aus, sodass die Stempel 6 bzw. 7 durch je eine Öffnung in der Kompressionsplatte 4 hindurch auf die Mammae 20 einwirken. Die Schwingungsbewegung wird mit Hilfe einer um eine vertikale Achse schwenkbar gelagerten Antriebsspule 10 bzw. 11 erzeugt, die von einem Wechselstrom durchflossen wird und dadurch im Hauptmagnetfeld B0 des Magneten durch die Pfeile 15 und 16 angedeuteten Schwenkbewegungen angeregt wird, die sich auf den Schwingungsapplikator 6, 8 bzw. 7, 9 übertragen.

Im folgenden wird die Funktion des Mammographie-Zusatzes in Verbindung mit einer MR-Elastographie-Untersuchung näher erläutert.

Nach der Lagerung der Patientin 40 entsprechend Fig. 1 wird die Kompressionsplatte 5 so verstellt, dass sich eine leichte Kompression ergibt, die ausreicht, um die Mammae zu fixieren, aber nicht so stark ist, dass diese nicht mehr elastisch verformbar sind. Durch diese Kompression werden die Mammae in Rechts-Links-Richtung verbreitert und in Fuß-Kopf-Richtung komprimiert.

Danach wird durch die Steuereinheit 35 ein Wechselstrom mit einer Frequenz von z. B. 200 Hz durch die Antriebsspulen 10, 11 vorgegeben, wodurch in den Mammae 20 mit dieser Frequenz vorwiegend longitudinale Wellen angeregt werden, die in die Mammae sehr gut eindringen können. Danach werden die zur Akquisition der benötigten MR-Daten erforderlichen MR-Sequenzen erzeugt. Dabei wird die Kernmagnetisierung in den Mammae in transversalen (zur Längsrichtung senkrechten) Schichten angeregt. Die Anregung der Kernmagnetisierung in einer einzelnen Schicht umfasst dabei die Erzeugung wenigstens eines Hochfrequenzimpulses durch die Hochfrequenzspule 32 und den Hochrequenzgenerator 34 in Verbindung mit einem von der Gradientenspulenanordnung 31 in Verbindung mit dem Generator 33 erzeugten und in Längsrichtung verlaufenden Gradienten des Magnetfeldes, so dass die Kernmagnetisierung in einer zur Längsrichtung senkrechten Schicht angeregt wird. Diese Kernmagnetisierung wird durch einen weiteren Magnetfeldgradienten in der Phase kodiert. Bevor das MR-Signal ausgelesen wird, wirkt auf den Untersuchungsbereich ein vorzugsweise sinusförmig verlaufendes periosiches Gradienten-Magnetfeld ein, dessen Periode durch die Steuereinrichtung 35 mit der Periode der mechanischen Wellen synchronisiert wird.

Dies wird für weitere Schichten wiederholt, bis die Mammae vollständig in parallelen Schichten erfasst sind.

Die Anregungen der Kernmagnetisierung in transversalen Schichten wird dann mit anderen Phasencodierungen wiederholt, bis genügend MR-Daten für eine vollständige Abbildung der Schicht vorhanden sind. Dieses Abbildungsverfahren wird dann mehrfach wiederholt, wobei die relative zeitliche Lage zwischen der mechanischen Schwingung und der sinusförmigen Gradientenschwingung geändert wird. Der gesamte Zyklus wird dann noch zweimal mit einer geänderten Orientierung des sinusförmig verlaufenden Magnetfeldes wiederholt. Es leuchtet ein, dass eine solche Untersuchung relativ viel Zeit benötigt.

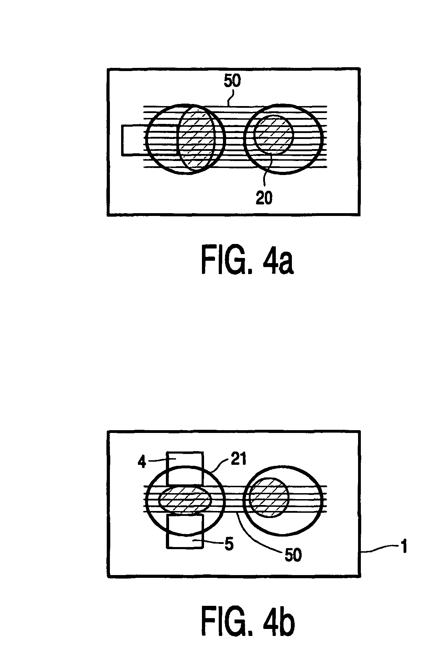

Die Untersuchungszeit hängt von der Zahl der transversalen Schichten ab, mit denen die Mammae erfasst werden können. Durch den erfindungsgemäßen Mammographie-Zusatz wird die Zahl der Schichten und damit die Untersuchungszeit verringert. Dies wird in den Fig. 4a und 4b veranschaulicht. Fig. 4a erläutert die Verhältnisse bei dem bekannten Mammographie-Zusatz nach der US-Appln 09/743659 (PHD 99-152). Die Mammae werden dabei in Rechts-Links-Richtung komprimiert und in Kopf-Fuß-Richtung vergrößert. Deshalb ist eine relativ große Zahl von Schichten 50 erforderlich, um die Mammae vollständig abzubilden.

Fig. 4b zeigt demgegenüber die Verhältnisse bei der Erfindung. Die Mammae werden in Kopf-Fuß-Richtung komprimiert und in Rechts-Links-Richtung verbreitert. Die Zahl der für eine vollständig Abbildung benötigte Schichten ist daher niedriger als bei dem bekannten Verfahren, und deshalb ist die für die Untersuchung benötigte Zeit entsprechend kürzer. Weil die Kompression dabei senkrecht zu den Schichtebenen erfolgt, bleibt die Form der Mamma in den einzelnen Schichtbildern weitgehend erhalten, während bei der in Richtung der Schicht erfolgenden Verformung nach Fig. 4a sich eine Darstellung ergibt, die von vielen Untersuchern abgelehnt wird.

Der Mammographie-Zusatz gemäß der Erfindung kann mit Vorteil auch für hochaufgelöste dynamische MR-Mammographie benutzt werden, bei der die Mammae mit Hilfe eines geeigneten, z. B. Gadolinium enthaltenden Kontrastmittels dargestellt werden. Auch hierbei ergibt sich eine verringerte Messzeit. Außerdem ist von Vorteil, dass durch die Kompressionsplatten 4 und 5 die Mammae fixiert sind, so dass Bewegungsartefakte vermieden werden.

Beide Verfahren - die dynamische MR-Mammographie und die MR-Elastographie - können bei einer Mamma-Untersuchung auch in Kombination miteinander angewandt werden. Dabei wird zunächst eine dynamische MR-Mammographie durchgeführt, und der dabei für die Diagnose als relevant erkannte Bereich wird anschließend mit Hilfe der MR-Elastographie untersucht.

Es ist nicht notwendig, dass die Kontaktflächen der Stempel 6, 7 genau senkrecht zu ihrer Schwingungsrichtung verlaufen. Damit durch die Schwingungserzeuger in den Mammae im wesentlichen longitudinale Wellen angeregt werden, ist es aber wichtig, dass die Kontaktflächen die Schwingungsrichtung unter einem von 0° verschiedenen Winkel schneiden.

## Patentansprüche

1. Mammographie-Zusatz für MR-Elastographie
mit einer Lagerungs-Einheit, die eine mit wenigstens einer Öffnung zur Aufnahme wenigstens einer Mamma versehene Stützfläche aufweist und
mit einer Schwingungserzeuger-Anordnung zur Erzeugung von mechanischen Schwingungen über wenigstens einen in Längsrichtung einer zu untersuchenden Patientin hin- und herschwingenden Schwingungsapplikator mit einer zu seiner Schwingungsrichtung zumindest näherungsweise senkrechten Kontaktfläche.

2. Mammographie-Zusatz nach Anspruch 1 mit einer ersten Kompressionsplatte, die auf der dem Schwingungsapplikator gegenüberliegenden Seite der Öffnung angeordnet ist und die in der Schwingungsrichtung des Schwingungsapplikators verstellbar ist.

3. Mammographie-Zusatz nach Anspruch 2 mit einer zweiten Kompressionsplatte, die auf der der ersten Kompressionsplatte gegenüberliegenden Seite der Öffnung angeordnet ist, die ortsfest bezüglich der Lagerungs-Einheit angeordnet ist und durch die hindurch der Schwingungsapplikator auf den Bereich zwischen den beiden Kompressionsplatten einwirkt.

4. Mammographie-Zusatz nach Anspruch 1 mit zwei Öffnungen für je eine Mamma, wobei im Bereich einer jeden Öffnung mindestens eine MR-Spule in einer zur Stützfläche parallelen Ebene zum Empfang von MR-Signalen und ein Schwingungsapplikator der Schwingungserzeuger-Anordnung vorgesehen sind.

5. MR-Gerät mit einem Mammographie-Zusatz nach Anspruch 1
mit einem den zeitlichen Verlauf von magnetischen Gradientenfeldern bestimmenden Generator und einer den Generator und den Mammographie-Zusatz steuernden Steuereinheit derart, dass die Mammographie-Zusatz erzeugten mechanischen Schwingungen und die magnetischen Gradientenfeldern synchron zueinander ablaufen.

## Claims

1. A mammography accessory for MR elastography which includes a positioning unit which is provided with a supporting surface in which at least one opening is formed so as to receive at least one of the mammae, and which includes an oscillation generating arrangement for generating mechanical oscillations via at least one oscillation applicator which performs a reciprocating motion in the longitudinal direction of a female patient to be examined and has a contact surface which extends at least approximately perpendicularly to its oscillation direction.

2. A mammography accessory as claimed in claim 1, which includes a first compression plate which is arranged at the side of the opening which faces the oscillation applicator and is adjustable in the oscillation direction of the oscillation applicator.

3. A mammography accessory as claimed in claim 2, which includes a second compression plate which is arranged at the side of the opening which faces the first compression plate, which is mounted so as to be stationary relative to the positioning unit and through which the oscillation applicator acts on the zone between the two compression plates.

4. A mammography accessory as claimed in claim 1, which includes two openings for a respective mamma, at the area of each opening there being provided at least one MR coil which is situated in a plane parallel to the supporting surface and serves to receive MR signals, and also an oscillation applicator which forms part of the oscillation generating arrangement.

5. An MR apparatus which includes a mammography accessory as claimed in claim 1, a generator which controls the variation in time of magnetic gradient fields, and a control unit which controls the generator and the mammography accessory in such a manner that the mechanical oscillations generated by the mammography accessory and the magnetic gradient fields are synchronized relative to one another.

## Revendications

1. Accessoire de mammographie pour élastographie RM avec une unité de logement qui présente une surface d'appui dotée d'au moins une ouverture pour le logement d'au moins un sein et avec un dispositif producteur d'oscillations pour la production d'oscillations mécaniques sur au moins un applicateur d'oscillations oscillant en va-et-vient dans la direction longitudinale d'une patiente à examiner avec une surface de contact perpendiculaire, du moins approximativement, à sa direction d'oscillation.

2. Accessoire de mammographie selon la revendication 1 avec une première plaque de compression qui est disposée sur la face de l'ouverture opposée à l'applicateur d'oscillations et qui est réglable dans la direction d'oscillation de l'applicateur d'oscillations.

3. Accessoire de mammographie selon la revendication 2 avec une deuxième plaque de compression qui est disposée sur la face de l'ouverture opposée à la première plaque de compression qui est fixée demeure par rapport à l'unité de logement et agit au travers de l'applicateur d'oscillations sur la zone située entre les deux plaques de compression.

4. Accessoire de mammographie selon la revendication 1 avec deux ouvertures pour chacune un sein, au moins une bobine RM étant prévue dans la zone de chaque ouverture dans un plan parallèle à la surface d'appui pour la réception de signaux RM et un applicateur d'oscillations du dispositif générateur d'oscillations.

5. Appareil RM avec un accessoire de mammographie selon la revendication 1 avec un générateur déterminant le tracé temporel de champ à gradient magnétique et une unité de commande commandant l'accessoire de mammographie de telle sorte que les oscillations mécaniques produites par l'accessoire de mammographie et les champs à gradient magnétiques évoluent réciproquement de manière synchrone.
